# EUROPEAN PATENT SPECIFICATION

(11) **EP 2 354 217 B1**
(45) Date of publication and mention of the grant of the patent: **02.09.2015**
(21) Application number: 10152517.8
(22) Date of filing: 03.02.2010
(51) Int. Cl.: B01F 11/00, C12M 1/32, C12M 1/12, C12M 1/34, B01L 3/00, C12Q 1/02, G01N 27/02, G01N 27/26, G01N 33/50, G01N 33/487

(54) **Integrated cultivation and measurement device for label-free detection and classification of cellular alterations, in particular for generation and characterisation of cell-spheroids, components and uses thereof**
Integrierte Kultivierungs- und Messvorrichtung für markierungsfreie Detektion und Klassifizierung von Zellveränderungen, speziell zur Erzeugung und Charakterisierung von Zellsphäroiden, Komponenten und Verwendungen damit
Dispositif de mesure et de culture intégré pour la détection sans marqueurs et la classification d'altérations cellulaires, en particulier pour la génération et la caractérisation de sphéroïdes cellulaires, composants et utilisations correspondantes

(43) Date of publication of application: 10.08.2011
(73) Proprietor: Universität Leipzig, 04109 Leipzig (DE)
(72) Inventor: Robitzki, Andrea, Prof. Dr., 68519 Viernheim (DE); Kurz, Randy, Dr., 04129 Leipzig (DE)
(74) Representative: Kailuweit & Uhlemann Patentanwälte Partnerschaft mbB

(56) References cited:
- WO-A-98/35021
- WO-A1-2009/137440
- WO-A2-02/29402
- DE-C1- 10 142 393
- US-A1- 2005 279 634
- US-A1- 2007 166 204
- US-A1- 2008 299 652
- KLOSS ET AL.: "Drug testing on 3D in vitro tissues trapped on a microcavity chip" LAB. CHIP, vol. 8, 11 April 2008 (2008-04-11), pages 879-884, XP002593125
- KLOSS ET AL.: "Microcavity array (MCA)-based biosensor chip for functional drug screening of 3D tissue models" BIOSENSORS AND BIOELECTRONICS, vol. 23, 12 January 2008 (2008-01-12), pages 1473-1480, XP002593126
- DATABASE WPI Week 200570 Thomson Scientific, London, GB; AN 2005-678836 XP002593127 -& JP 2005 261365 A (IWAMOTO ET AL.) 29 September 2005 (2005-09-29)

## Description

The present invention relates to a device and a method for integrated cultivation of cells, cell-spheroids and tissues and a non-destructing label-free characterisation of physiological events of these cells, in particular for generation and characterisation of cell-spheroids.

The use of living cells as biosensors is important because it gives the functional information of the effect of a stimulus. Only use of living cells helps to solve those various questions, such as the effect of an active pharmaceutical ingredient on a given physiological system, i.e. if a a substance modulates unknown aspects of the cellular metabolism or if it has a toxic effects on mammalian cells (L. Bousse, Sensors and Actuators B 34 (1996) pp. 270-275). Mammalian cell based biosensors were also developed for fast (high throughput) and economic determination of effects of active pharmaceutical ingredients in the preclinical phase of drug approval. At the moment mammalian cell based-biosensors often use 2D cell monolayer as recognition elements, but the influence of active pharmaceutical ingredients on complex cell-cell interactions can not be determined with sufficient reliability. The diffusion or assimilation of an active pharmaceutical ingredient inside of a tissue cannot be determined with 2D cell monolayer sensors. The answer of cell monolayer to a stimulus is not comparable with living tissue, because the 2D cell monolayer culture is a very artificial system and does not exist *in vivo.*

A better *in vitro* model for an *in vivo* system is a 3D cell culture system. It avoids the disadvantages of 2D cell monolayer system and has better inter- and intracellular interactions. Also the differentiation of stem cells to differentiated cells needs a 3D culture system. Accepted 3D cell cultures are so-called spheroids. Spheroids are three-dimensional, globular aggregates of several thousand cells.

The use of three-dimensional cell-spheroids for the screening of active pharmaceutical ingredients and their effects on the cells offers the possibility to monitor effects in a more tissue-like environment than an environment provided by conventional two-dimensional cell monolayers.

DE19953424B4 describes a method and a device for an automatical production of homogeneous spheroids. The spheroids are generated in a (bio)reactor, separated via a culture medium flow and can be characterised individually. A disadvantage is the separation of the generation of spheroids and the further characterisation or treatment with active pharmaceutical ingredients.

A device and method for characterisation of whole spheroids is described in EP 1221032 B1. The spheroids are transferred in a capillary and the impedance is measured between two, on opposite exits of the capillary situated, electrodes. The positioning of the spheroids is controlled by applying a proper pressure on the medium in the capillary. In this system also the cultivation and the characterisation of the spheroids is separated and the spheroids have to be moved manually between the two stages.

DE10142393C1 describes the characterisation of spheroids via a multielectrode arrangement with simultaneous measurement of the impedance and field potential signals. The system needs also a manual transfer of spheroids from a culture system to the measurement system. Another disadvantage is the positioning of the spheroids in the electrode arrangement with a vacuum pressure that has to be fine-tuned for every spheroid diameter.

Kloß et al. (Lab Chip, 2008, 8, 879-884, Biosensors and Bioelectronics 23, 2008, 1473-1480) describe a biosensor chip for impedance measurement and extracellular recording for functional drug screening of 3D tissues models.

The characterisation of the differentiation of stem cells to differentiated cells like cardiomyocytes is used in the embryonic stem cell test. Active pharmaceutical ingredients can be characterised concerning to their embryotoxicity (A. Seiler et al. Reproductive Toxicology 18 (2004) pp. 231-240). Molecular markers for cytotoxicity and apoptosis as well as beating characteristics and/or contractility of the differentiated cardiomyocytes are used as parameters of the embryotoxicity (Genschow et al. In Vitro Molelcuar Toxicology 12 (2000) pp. 51-65 and Spielmann et al. Alternatives to Laboratory Animals 29 (2001) pp. 301-303). These methods have several disadvantages. The generation of the stem cell spheroids occurs via manual pipetting with the manual hanging drop method, also the separation of the spheroids in different culture chambers takes place manually. The counting of the beating of the cardiomyocytes and the molecular characterisation are time consuming, cumbersome methods that require high technical expertise.

Up to now there exists no system and method that would allow the automated generation, cultivation and characterisation of spheroids for a high parallel screening of possible effects of active pharmaceutical ingredients or an automated system for an embryotoxicity screening. For reproducible and significant results about the influence and side effects of potential active pharmaceutical ingredients it is necessary to minimize the human influence to the measurement system, and to use an automated generation and label-free characterisation of multiple spheroids in a highly parallel system.

The object of the present invention is therefore to provide an integrated cultivation and measurement device and a method for label-free detection and classification of cellular alterations, in particular a device that allows an automated generation and characterisation of cell-spheroids, components and uses thereof.

This object is solved by a device according to claim 1 comprising:
a) a mounting device for a cultivation chamber plate with several culture reservoirs, whereas the bottom of each culture reservoir forms a microcavity and each microcavity features microelectrodes on the microcavity walls and whereas the mounting device has contacts for the microelectrodes,
b) an amplifier board with an interface, linked with the contacts for the microelectrodes in the mounting device,
c) a rotary shaker, on which the amplifier board and the mounting device for the cultivation chamber plate are placed, and
d) a recording, analyzing and central controlling unit (control unit) that is linked with the amplifier board and the rotary shaker.

The device comprises a core unit comprising a rotary shaker, on which the amplifier and mounting device for the cultivation chamber plate are placed. Advantageously, this core unit can be placed into a standard cell culture incubator.

An important component of the system is the cultivation chamber plate in which the culture reservoirs are arranged. The cultivation chamber plate is preferably a microtiter plate that has the dimensions of a standard microtiter plate with multiple wells as culture reservoirs for parallel cultivation. Preferably, each cultivation chamber plate has 1 to 1000 wells, preferably 24, 48, 96 or 384 wells. The use of the microtiter plate format allows the linking of this device with other laboratory automation devices like pipetting roboters that inject active pharmaceutical ingredients to the culture reservoirs or change the culture medium. Every culture reservoir features a microcavity, preferably with an edge length of 100 to 500 µm, that is implemented in the bottom of the culture reservoir. Several microelectrodes (at least two, preferably 4 to 12) are situated at the side walls of the microcavity and connected with contact pads at the outside of the cultivation chamber plate. The microcavities are preferably designed as inverted truncated pyramidal structures with preferably 4 to 8 sides and preferably one microelectrode on every side or as inverted truncated cone structure with preferably multiple microelectrodes on the wall.

Preferably the microelectrodes are positioned in the upper half of the microcavity wall and in the middle third of the every microcavity side. The geometry of the microelectrodes preferably is rectangular or planar, but can also be carried out as planar meandering microelectrode or as 3D spike that protrudes in the cavity.

Furthermore, every culture reservoir preferably features a reference electrode for field potential measurements. The microelectrodes are preferably bonded with contact pads at the side of the cultivation chamber plate. When the cultivation chamber plate is placed into the mounting device, the contact pads are connected with the amplifier board. Multiple amplifier boards each comprising at least one mounting device for at least one cultivation chamber plate can be placed on the rotary shaker.

Preferably the cultivation chamber plate is made of plastic or glass and the microcavities are made via laser ablation, or micromolding. In a preferred embodiment the cultivation chamber plate is transparent to allow a multimodal imaging of cells, tissue or cell-spheroids.

Preferably the culture reservoirs have a physically and/or (bio)chemically functionalized surface. Preferred but not limiting examples for such a functionalization are selected from coating with poly-L-Lysine, heparin and cellular matrix components (such as collagen, fibronectin, elastin, hyaluronic acid and proteoglycans), silanizing of the surface, roughness modifications by laser ablation or micromolding (like grooves) or UV-radiation conditioning.

In a preferred embodiment the functionalized surface of the culture reservoirs and/or the electrode surface is nanostructured, i.e. in the form of a nanocolumnar electrode surface (as nanocolumnar platin electrode), or nanoporous surfaces, like a functionalized nanoporous silicon surface.
The cultivation chamber plate can be designed to be a reusable part or single use part. After use the cultivation chamber plate in the amplifier board can be exchanged. The cultivation chamber plate can be sold together with the device or as a separate component.

The contact pads of the cultivation chamber plate are connected via the mounting device with the amplifier board circuit. The mounting device fixes the cultivation chamber plate onto the rotary shaker. The mounting device is preferably an integral part of the rotary shaker or fixed onto it. The mounting device contains a holding fixture, e. g. in form of a slot or edges and connects the contact pads of the cultivation chamber plate with the amplifier board circuit. The mounting device can be constructed to hold one or several cultivation chamber plates.

In a preferred embodiment the mounting device for the cultivation chamber plate is coupled with a microlaser manipulation system, allowing manipulation of the cells, cell-spheriods or tissue.

The amplifier board preferably features an integrated multiplexer, impedance analyzer and an amplifier with analog/digital converter. The multiplexer allows switching of every microelectrode in a circuit with the impedance analyzer. The amplifier board is devised such that the impedance between the microelectrodes and the field potential at the microelectrodes of the cultivation chamber can be measured serially or simultaneously.

The impedance spectra of an object e.g. a cell-spheroid in the microcavity is preferably determined by applying an alternating current (preferably with a logarithmic sweep within a frequency range of 1 kHz to 1 MHz) to the microelectrodes and measuring the according current. The amplifier with an analog to digital converter can also be switched to every microelectrode and allows the recording of the field potential of every microelectrode. A reference electrode for the field potential recordings is preferably situated in every culture reservoir. The amplifier board is managed by the control unit and this unit also records the measured data from the amplifier board for online analysis.
A central part of the device is the rotary shaker. The amplifier board with the mounting device for the cultivation chamber plate is situated on the rotary shaker and is preferably orbital moved. Thus, the rotary shaker is preferably an orbital shaker. The rotary shaker is managed by the control unit. Preferably, said control unit also records the shaker status parameter. In every culture reservoir preferably exactly one cell-spheroid is generated by applying optimized settings, such as rotation parameters, like shaker orbital radius, constant or alternating rotation speed, managed by the control unit. The rotation parameters can be adjusted for the generation of spheroids from every cell type.

The cell-spheroids generated in a device according to the invention are preferably cultivated in rest (statically, without rotation) or under constant rotation of the culture reservoirs. Spheroids can be generated from different cell types like primary cells (cardiomyocytes, retinal cells etc.), cell lines (tumour cells) and stem cells. The cell-spheroids generated by the device according to the invention comprise multiple cells that form a spheroid structure. The number of cells and diameter of the cell-spheroid depends on the number of cells inserted into the microcavity and the rotation speed. Advantageously, in the device according to invention a single cell-spheroid per microcavity is generated, independent of the number of cells inserted into said microcavity.

The monitoring of the cellular events is based on the measurement of the bioelectrical properties of electrogenic cells like cardiomyocytes and non-electrical parameters via bioimpedance spectroscopy. Preferably said properties are measured simultaneously. For characterisation, the spheroids are positioned in the microcavities, preferably positioning is carried out automatically, via the controlled (preferably orbital) rotation of the cultivation chamber plate. The right positioning of the spheroids in the microcavities is preferably controlled via rising of the measured impedance between two opposite electrodes situated on the walls of the microcavity. The characterisation of the spheroids is preferably carried out via determination of electrical and electrophysiological parameters. For that purpose preferably the impedance between different electrodes and the field potential on every electrode is measured and recorded. The impedance spectra is preferably measured in the so-called β -dispersion in a range of 100 Hz to 10 MHz and gives information about charge changes on the membrane and subsequent cellular properties like apoptosis, necrosis, cell-cell-interactions, proliferation and receptor activation pathways. This impedance spectrum is preferably measured between every electrode pair of the microcavity and gives detailed information about the spheroid properties. The impedance spectra are measured with an impedance analyser that is preferably situated in the amplifier board and managed by the control unit. The measurement electrodes are preferably switched with integrated multiplexers that are also managed by the control unit. Alternatively the impedance is measured in the high frequency range from 100 kHz to 10 GHz.

Another characterisation possibility is the measurement of the bioelectrical properties as field potentials at every electrode of the microcavity. This field potential data allows the characterisation of electrogenic cells like cardiomyocytes or neuronal cells. The amount and rate of the field potential spikes gives information about properties of these cells. The beginning contraction rates of cardiomyocytes differentiated from stem cells and their modulation can also be detected and can be used for an embryotoxicity test. The field potential signals have to be amplified with an integrated amplifier situated in the amplifier board and were recorded with the control unit. After every measurement the spheroids are removed from the microcavities via rotation and move freely in the culture reservoir.

The preferred use of transparent materials for the cultivation chamber plate like plastic or glass, and transparent electrode material like indium tin oxide (ITO) allows an additional multimodal imaging of the spheroids that has the advantage that it allows further characterisation of the spheroids.

The recording, analyzing and central controlling unit (also referred to herein as control unit) manages the complete device and can be pre-programmed. The control unit controls the rotation parameters of the rotary shaker during the generation of the spheroids and at the cultivation and measurement period. Preferably, the control unit also manages the amplifier board with the multiplexer, the impedance analyser and the amplifier for the field potential measurement and records the impedance and field potential data. Advantageously, a standard personal computer (PC) can be used as control unit. The control unit preferably allows linking multiple amplifier boards, e. g. via an adapter.

The advantage of the device according to the invention is that it allows the complete automated generation and non-destructive characterisation of spheroids along a measurement period. The use of three-dimensional cell-spheroids for the screening of active pharmaceutical ingredients and their effects on the cells offers the possibility to monitor effects in a more tissue-like environment as conventional two-dimensional cell monolayer provides. The device allows the automatic generation and cultivation of three-dimensional cell-spheroids derived from single cells and the high content monitoring of cell-cell connections, apoptosis, necrosis, proliferation and differentiation in this cell-spheroids and their changes related to potential active pharmaceutical ingredients.

Preferably all parts of the device except for the control unit are situated in a cell culture incubator during function.

Another object of the invention is the use of the device and the cultivation chamber plate according to the invention for the cultivation of cells or tissues, the label-free detection and classification of cellular alterations, in particular for generation of cell-spheroids and monitoring the condition of the cell-spheroids.

The invention also comprises a method for label-free detection and classification of cellular alterations, in particular for generation and characterisation of cell-spheroids and monitoring the condition of the cell-spheroids in real time, comprising:
a) providing a device and a cultivation chamber plate according to the invention,
b) introducing cells, cell-spheroids or a tissue sample in the culture reservoirs of the cultivation chamber plate,
c) positioning of the cells, cell-spheroids or a tissue sample in the microcavities of the culture reservoirs by the movement of the rotary shaker,
d) determining the impedance of the cells, cell-spheroids or tissue sample between two microelectrodes and/or electrogenic activity of the cells, cell-spheroids or tissue sample on every electrode and their changes during the cultivation and between different culture reservoirs.

In step c) the cells, cell-spheroids or a tissue sample are advantageously positioned in the microcavity of the corresponding culture reservoir by the movement of the rotary shaker. After determining of the parameters in step d) the cell, cell-spheroid or tissue samples are preferably moved out of the bottom of the microcavity (but not out of the culture reservoir) by the movement of the rotary shaker. This movement advantageously prevents adherence of the cells to the surface of the microcavity.

When the invention is applied to generate cell-spheroids the method of the invention comprises an additional step b') (between step b) and c)). Thus in this case the method of the invention comprises:
a) providing a device and a cultivation chamber plate according to the invention,
b) introducing cells in the culture reservoirs of the cultivation chamber plate,
b') generation of cell-spheroids managed by the control unit via cell-specific shaking programmes and
c) positioning the cell-spheroids in the microcavities of the culture reservoirs,
d) determining the impedance of the tissue sample between two microelectrodes and/or electrogenic activity of the cell-spheroid on every electrode and their changes during the cultivation and between different culture reservoirs.

In a preferred embodiment stem cells are used for the generation of cell-spheroids. Here, the generation of the cell-spheroids by using the invention preferably initiates a differentiation of the stem cells. Preferably, stem cells are obtained without destruction of human embryos. Preferred stem-cells are non-human embryonic stem cells, human induced pluripotent stem cells, somatic stem cells or cord blood stem cells. Preferably, the stem cells differentiate to cardiomyocytes and the modulator is a known or suspected modulator of the electrophysiological properties of the cardiomyocytes and/or impacts the differentiation of the stem cells.

In another embodiment the cells are isolated from tissue samples.

When the invention is applied on tissue samples (without prior isolation of single cells) the method of the invention comprises the steps:
a) providing a device and a cultivation chamber plate according to the invention,
b) introducing a tissue sample in the culture reservoirs of the cultivation chamber plate,
c) positioning the tissue sample in the microcavities of the culture reservoirs,
d) determining the impedance of the tissue sample between two microelectrodes and/or electrogenic activity of the cell-spheroid on every electrode and their changes during the cultivation and between different culture reservoirs.

The positioning in step c) and determining of spheroid parameters in step d) can be performed at an arbitrary time, like every hour, every 6 hours or every day

In a preferred way to carry out the method according to the invention a known or suspected modulator of the cell condition in subset of culture wells is introduced after step b.) or b.') or after step d.). Particularly in the latter case, steps c.) and d.) are iteratively repeated.

Preferably the impedance is determined between all electrodes to achieve a spatial resolution of the impedance in the cell-spheroid.

The modulator is a substance which has a known biological effect or a substance to be tested if it has such an effect.

The modulator is preferably a toxic or cytostatic or anti-toxic substance or a substance to be tested if it has a toxic or cytostatic or anti-toxic effect.

Alternatively or additionally, the modulator is a pharmaceutical active ingredient.

Alternatively or additionally, the modulator has an apoptotic or necrotic effect, or an anti-apoptotic or anti-necrotic effect, or a substance to be tested if it has an apoptotic or necrotic effect, or an anti-apoptotic or anti-necrotic effect.

The invention is further illustrated by the following figures and a specific embodiment, without being limited to these:

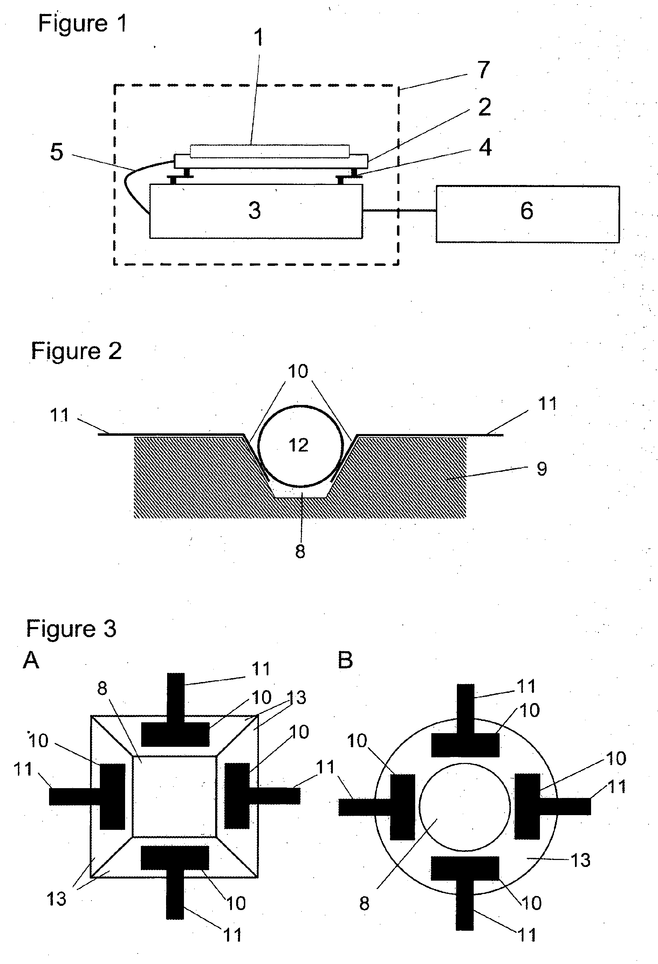

**Figure 1****:** Shown is the schematic layout of the integrated cultivation and measurement device with cultivation chamber plate **1,** amplifier board **2,** rotary shaker **3** with rotation axis **4,** connection cable from amplifier board to the rotary shaker **5** and the central control unit **6.** All parts except for the central control unit are situated in a cell culture incubator **7.**
**Figure 2****:** Shown is a scheme of the cross section through a microcavity **8** with the cultivation chamber plate material **9,** the microelectrodes **10** with interconnects (with contact pad) **11** and a cell-spheroid **12.**
**Figure 3****:** Shown are the top views of two embodiments of a microcavity **8.** The microcavity is realized **a)** as inverted truncated pyramidal structure with 4 sides and **b)** as inverted truncated cone structure. Both microcavities feature 4 microelectrodes **10** on the microcavity wall **13** with interconnects (with contact pad) **11.**

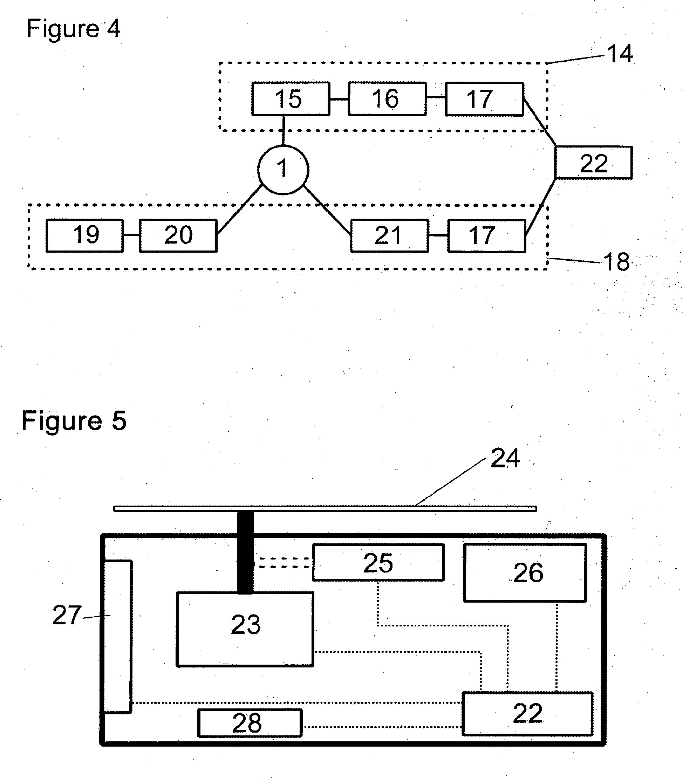

**Figure 4****:** The functions of the amplifier board **2** are illustrated as a block diagram with the connection to the cultivation chamber plate **1.** The field potential measurement part **14** features a noise filter **15** an amplifier **16** and an analog/digital converter **17.** The impedance measurement part **18** features a signal generator **19,** a multiplexer **20,** a measuring transducer **21** and an analog/digital converter **17.** Both parts are operated by a microcontroller **22.**
**Figure 5****:** Shown is the schematic view of the elements of the rotary shaker **3.** The rotary shaker features a geared motor **23** with an adapter for the amplifier board **24,** a photointerruptor **25** for motor speed control, a digital thermo sensor **26,** a fan **27** and an acceleration sensor **28** for tilt control. These elements are controlled by a microcontroller **22** with an interconnection to the central control unit **6.**

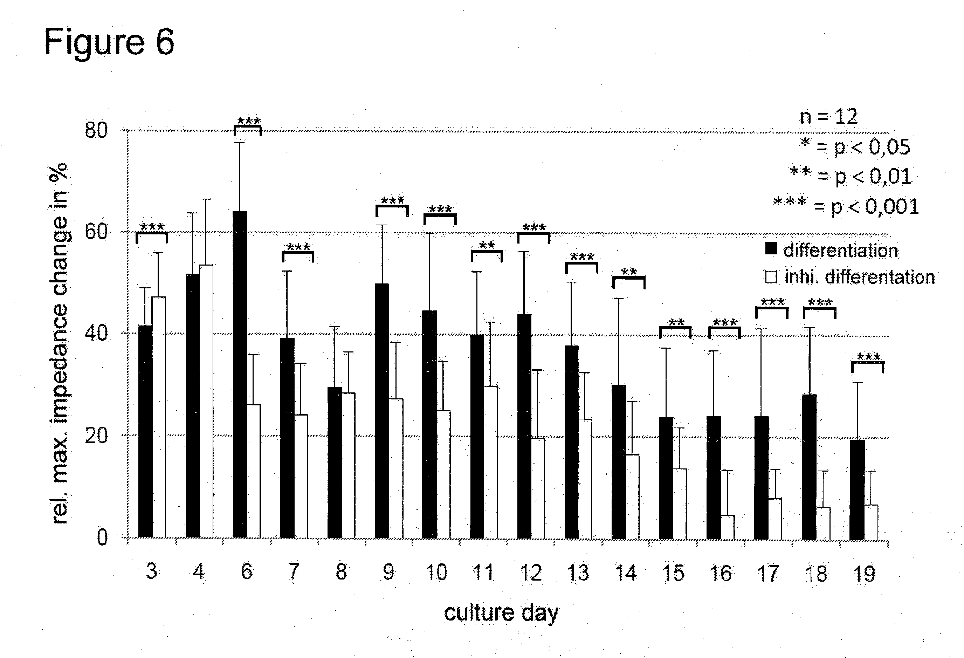

**Figure 6****:** Shown is the relative maximal impedance change in percent of mouse embryonal stem cells over 19 culture days measured in a 400 µm wide truncated pyramidal microcavity with 4 sides. 12 cell-spheroids were cultured on the rotary shaker in cardiac differentiation media and 12 cell-spheroids were cultured on the rotary shaker in inhibiting differentiation media. The differentiated cell-spheroids had significant higher impedance than the cell-spheroids with inhibited differentiation.

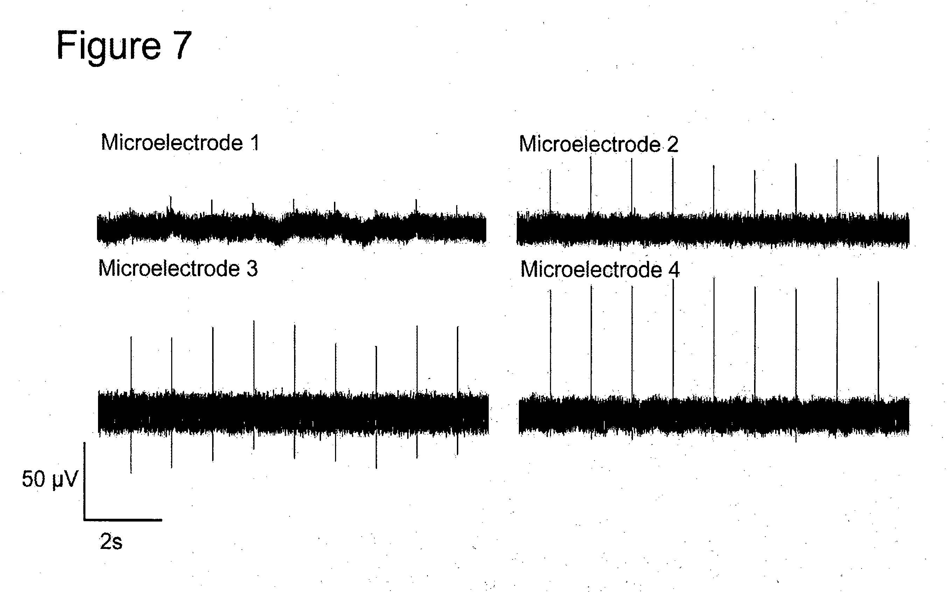

**Figure 7****:** Shown are the field potentials of the 4 microelectrodes of a microcavity with a mouse embryonal stem cell derived cardiomyocyte cell-spheroid. The considerable field
potential spikes of the microelectrodes 2, 3 and 4 correlates clearly with the visible contractions of the cardiomyocytes cell-spheroids.

### Embodiment

The integrated cell-spheroid generation and sensing device consists of a rotary shaker **3** that is situated in a cell culture incubator **7** with high humidity (Fig. 1). Hence the geared motor **23** and all electronic components are encapsulated (Fig. 1 and Fig. 5). The rotary shaker **3** works in an orbital mode with a rotation diameter of 6 cm that is defined by the rotation axis **4.** The number of revolutions of the geared motor **23** is continuously adjustable from 10 to 150 revolutions per minute for predefined and free selectable rotation protocols. As rotary shaker **3** status parameters, the tilt of the shaker is controlled by an acceleration sensor **28** and the number of revolutions is logged by a photointerruptor **25.** To avoid a heat accumulation above the geared motor a fan **27** is situated at the side wall of the rotary shaker. The speed of the fan is controlled by the microcontroller **22** and verified with a digital thermo sensor **26.** The status parameter of the rotary shaker **3** and the geared motor **23** are also controlled by the microcontroller **22** that is connected via USB or ZigBee wireless network to a PC as central control unit **6.** As good laboratory practice (GLP) qualification the PC records the rotation and status parameters of the rotary shaker **3.** The rotary shaker **3** has an adapter for the amplifier board **24.**

The amplifier board **2** has an interface for the cultivation chamber plate **1** in the standardized 96 well microtiter plate format. The connection to the contact pads of the cultivation chamber plate **1** is carried out with gold contact pins. The amplifier board **2** comprises an impedance measurement part **18,** a field potential measurement part **14** and a microcontroller **22** (Fig. 4). With a signal generator **19,** a multiplexer **20** and a measuring transducer **21** the impedance between two arbitrary microelectrodes **10** of an arbitrary microcavity **8** is determined via the contact pins. These components are controlled via an analog/digital converter **17** by a microcontroller **22** in the amplifier board **2** that is connected via USB or ZigBee wireless network to the controlling PC **6.** The field potential of every microelectrode **10** is measured with a noise filter **15** and an amplifier **16** against a central reference electrode that is located on the bottom of every culture chamber and connected to the amplifier board **2** via a contact pin. The data of the amplifier **16** is recorded via an analog/digital converter **17** by the microcontroller **22.** The circuitry of the amplifier board **2** is carried out in standard printed circuit board technology.

The cultivation chamber plate **1** consists of two parts that are joined together by adhesive bonding. The cultivation chambers in the standardized 96 well microtiter format are formed by the upper part. The microcavities **8** are situated on the lower part of the cultivation chamber plate **1.** A third part is a lid that is situated on the upper part of the cultivation chamber. All parts are fabricated by injection molding of a transparent biocompatible polycarbonate copolymer.

The microcavities **8** are carried out as inverted truncated pyramidal structure with 4 sides and an upper side length of 400 µm (Fig. 3A). A rectangular microelectrode **10,** consisting of indium tin oxide, with a side length of 200 µm and 20 µm is situated on every microcavity side. Isolated conductive paths **11** connect the microelectrodes with the contact pads on outside of the cultivation chamber plate **1.** The manufacturing of the microelectrodes **10,** the conductive paths (Interconnect with contact pad) **11** and the reference electrode is done by photolithography. The protection of the polycarbonate against the solvent of the photoresists is accomplished by a HF-sputtered 30 nm thick SiO₂ layer. The microelectrodes **10** and the reference electrode are made of indium tin oxide a transparent conducting material. All parts of the cultivation chamber plate can be sterilized via autoclaving.

The PC as central as central control unit **6** is connected with the rotary shaker **3** and the amplifier board **2** via USB (Universal Serial Bus) or ZigBee wireless network. The experiment setup with rotation parameter, measurement parameters and measurements points and can be preprogrammed and subsequent the carried out automatically. The experiment parameters and the measured data are saved as raw data on the PC in compliance with the good laboratory practice (GLP).

The system can be used as a quality control system for differentiation of embryonic stem cells in cardiomyocytes. For the forming of a cell-spheroid **12** 1000 embryonic stem cells were transferred with 100 µl culture medium into every culture chamber. In a cell culture incubator a single cell-spheroid was constituted after 3 days of rotation on the rotary shaker with 72 revolutions per minute in every culture chamber. The cell-spheroids could be cultivated on the rotary shaker for further 16 days.

The characterisation of cell-spheroids **12** is accomplished by positioning of a cell-spheroid in a microcavity **8** and measuring the impedance between two microelectrodes **10** of the microcavity **8** in a frequency range from 50 to 1000 kHz (Fig. 2). Afterwards the relative impedance change in percent was calculated from the impedance spectra without a cell-spheroid **12** in microcavity **8** and the impedance spectra with a cell-spheroid **12** the same micro cavity **8.**

The inventors showed that during 16 days of differentiation after formation of cell-spheroids, the embryonic stem cell-spheroids **12** in the culture medium showed significant higher maximum of the relative impedance change compared to cell-spheroids in a medium that the differentiation inhibit (Fig. 6). It was demonstrated that the embryonic stem cell-spheroids started to contract at culture day 10. The contractions and by this, the action potentials of the cardiomyocytes of the differentiated stem cell-spheroids could be shown via field potential measurements of the microelectrodes as characteristic spikes (Fig. 7). Cell-spheroids with an inhibited differentiation show no contractions and consequently no spikes in the field potential data.

The following reference numbers are used in the drawings and the claims:
- **1**: Cultivation chamber plate
- **2**: Amplifier board
- **3**: Rotary shaker
- **4**: Rotation axis
- **5**: Connection cable
- **6**: Control unit
- **7**: Cell culture incubator
- **8**: Microcavity
- **9**: Cultivation chamber plate material
- **10**: Microelectrode
- **11**: Interconnect with contact pad
- **12**: Cell-spheroid
- **13**: Microcavity wall
- **14**: Field potential measurement part
- **15**: Noise filter
- **16**: Amplifier
- **17**: Analog/digital converter
- **18**: Impedance measurement part
- **19**: Signal generator
- **20**: Multiplexer
- **21**: Measuring transducer
- **22**: Microcontroller
- **23**: Geared motor
- **24**: Adapter for amplifier board
- **25**: Photointerruptor
- **26**: Digital thermo sensor
- **27**: Fan
- **28**: Acceleration sensor

## Claims

1. An integrated cultivation and measurement device for label-free detection and classification of cellular alterations, for generation and characterisation of cell-spheroids and monitoring the condition of the cell-spheroids in real time, comprising
a) a mounting device for a cultivation chamber plate (1), wherein the cultivation chamber plate (1) has several culture reservoirs, wherein the bottom of each culture reservoir forms a microcavity (8) and each microcavity (8) features microelectrodes (10) on the microcavity walls (13) and wherein the mounting device has contacts for the microelectrodes,
b) an amplifier board (2) linked with the contacts for the microelectrodes (10) in the mounting device,
c) a rotary shaker (3), on which the amplifier board (2) and the mounting device for the cultivation chamber plate (1) are placed, and
d) a control unit (6), that is linked with the amplifier board (2) and the rotary shaker (3), wherein the control unit (6) allows recording, analyzing of data and controlling the movement of the rotary shaker (3).

2. A device according to claim 1 devised such that the positioning of cell-spheroids in the microcavities (8) is managed automatically by the control unit (6) via movement of the amplifier board (2) and cultivation chamber plate (1) by the rotary shaker (3).

3. A device according to claim 1 or 2 further comprising different adjustable rotation protocols for the rotary shaker (3) for generation of cell-spheroids from different types of cells and tissues managed by the control unit (6).

4. A device according to one of the claims 1 to 3, wherein the mounting device for the cultivation chamber plate (1) is coupled with a microlaser manipulation system.

5. Use of a cultivation chamber plate (1) with several culture reservoirs in a device according to one of the claims 1 to 4, wherein the bottom of each culture reservoir forms a microcavity (8) and each microcavity (8) features microelectrodes (10) on the microcavity walls (13).

6. Use according to claim 5, wherein the microcavities (8) of the cultivation chamber plate (1) are designed as
a.) an inverted truncated pyramidal structure, preferably with 4 - 8 sides and one microelectrode (10) on every side, or
b.) an inverted truncated cone microcavity, preferably with 4 - 12 microelectrodes (10) on the wall (13).

7. Use according to claim 5 or 6, wherein the cultivation chamber plate (1) features an integrated circuit for pre-processing of the measured data.

8. Use of a device according to any of the claims 1 to 5 or a cultivation chamber plate as defined in any of the claims 5 to 7 for the cultivation of cells or tissues, the label-free detection and classification of cellular alterations, in particular for generation of cell-spheroids and monitoring the condition of the cell-spheroids

9. A method for label-free detection and classification of cellular alterations, in particular for generation and characterisation of cell-spheroids and monitoring the condition of the cell-spheroids in real time, comprising:
a) providing a device according to one of the claims 1 to 4 and a cultivation chamber plate (1) as defined in one of the claims 5 to 7,
b) introducing cells, cell-spheroids or a tissue sample in the culture reservoirs of the cultivation chamber plate (1),
c) positioning of the cells, cell-spheroids or a tissue sample in the microcavities (8) of the cultivation chamber plate (1) by the movement of the rotary shaker (3),
d) determining the impedance of the cells, cell-spheroids or tissue sample between two microelectrodes (10) and/or electrogenic activity of the cells, cell-spheroids or tissue sample on every microelectrode (10) and their changes during the cultivation and between different culture reservoirs.

10. A method according to claim 9, for generation and characterisation of cell-spheroids and monitoring the condition of the cell-spheroids in real time, comprising:
- in step b) cells are introduced in the culture reservoirs of the cultivation chamber plate (1),
- in an additional step b') cell-spheroids are generated by movements of the rotary shaker (3) and managed by the control unit (6) via cell-specific shaking programmes and
- in step c) the cell-spheroids are positioned in the microcavities of the culture reservoirs,
- in step d) the impedance of the tissue sample between two microelectrodes (10) and/or electrogenic activity of the cell-spheroid on every microelectrode (10) and their changes during the cultivation and between different culture reservoirs is determined.

11. A method according to claim 9 or 10, wherein a known or suspected Modulator of the cell condition in subset of culture reservoirs is introduced after step b), b')or after step d).

12. A method according to claim 9 or 10, wherein a known or suspected modulator of the cell condition in subset of culture reservoirs is introduced after step d), further comprising iterative repeating of steps c) and d).

13. A method according to one of the claims 9 to 12 wherein the modulator is a possible toxic or cytostatic substance and/or a pharmaceutical active ingredient.

14. A method according to claim one of the claims 10 to 13, wherein the cells are stem cells and the generation of the cell-spheroids initiates a differentiation of the stem cells.

15. A method according to claim 14, wherein the stem cells differentiate to cardiomyocytes and the modulator is a known or suspected modulator of the electrophysiological properties of the cardiomyocytes.

## Patentansprüche

1. Integrierte Kultivierungs- und Messvorrichtung zum markerfreien Erkennen und Klassifizieren von Zellveränderungen, zum Erstellen und Charakterisieren von Zell-Sphäroiden und zum Überwachen der Zell-Sphäroiden in Echtzeit, Folgendes umfassend:
a) eine Halterungsvorrichtung für eine Kultivierungskammerplatte (1), wobei die Kultivierungskammerplatte (1) mehrere Kulturreservoirs aufweist, wobei der Boden eines jeden Kulturreservoirs eine Mikrokavität (8) ausbildet und jede Mikrokavität (8) Mikroelektroden (10) an der Mikrokavitätwand (13) aufweist und wobei die Halterungsvorrichtung Kontakte für die Mikroelektroden aufweist,
b) eine Verstärkerplatte (2), verbunden mit den Kontakten für die Mikroelektroden (10) in der Halterungsvorrichtung,
c) einen Orbitalschüttler (3), auf dem die Verstärkerplatte (2) und die Halterungsvorrichtung für die Kultivierungskammerplatte (1) angeordnet sind, und
d) eine Steuereinheit (6), die mit der Verstärkerplatte (2) und dem Orbitalschüttler (3) verbunden ist, wobei die Steuervorrichtung (6) das Aufzeichnen, das Analysieren von Daten und das Steuern der Bewegung des Orbitalschüttlers (3) ermöglicht.

2. Vorrichtung nach Anspruch 1, derart gestaltet, dass das Anordnen von Zell-Sphäroiden in den Mikrokavitäten (8) von der Steuervorrichtung (6) über die Bewegung der Verstärkerplatte (2) und der Kultivierungskammerplatte (1) durch den Orbitalschüttler (3) automatisch gesteuert wird.

3. Vorrichtung nach Anspruch 1 oder 2, ferner umfassend verschiedene einstellbare Drehprotokolle für den Orbitalschüttler (3) zum durch die Steuervorrichtung (6) verwalteten Erzeugen von Zell-Sphäroiden aus Zellen und Geweben verschiedener Typen.

4. Vorrichtung nach einem der Ansprüche 1 bis 3, wobei die Halterungsvorrichtung für die Kultivierungskammerplatte (1) mit einem Mikrolaser-Manipulierungssystem gekoppelt ist.

5. Gebrauch einer Kultivierungskammerplatte (1) mit mehreren Kulturreservoirs in einer Vorrichtung nach einem der Ansprüche 1 bis 4, wobei der Boden eines jeden Kulturreservoirs eine Mikrokavität (8) ausbildet und jede Mikrokavität (8) Mikroelektroden (10) an den Mikrokavitätwänden (13) aufweist.

6. Gebrauch nach Anspruch 5, wobei die Mikrokavitäten (8) der Kultivierungskammerplatte (1) gestaltet sind als:
a.) umgekehrte Pyramidenstumpfanordnung, vorzugsweise mit 4-8 Seiten und einer Mikroelektrode (10) auf jeder Seite, oder
b.) umgekehrte Kegelstumpf-Mikrokavität, vorzugsweise mit 4-12 Mikroelektroden (10) an der Wand (13).

7. Gebrauch nach Anspruch 5 oder 6, wobei die Kultivierungskammerplatte (1) einen integrierten Schaltkreis zum Vorverarbeiten der gemessenen Daten aufweist.

8. Gebrauch einer Vorrichtung nach Anspruch 1 bis 5 oder einer Kultivierungskammerplatte nach einem der Ansprüche 5 bis 7 zum Kultivieren von Zellen oder Gewebe, zum markerfreien Erkennen und Klassifizieren von Zelländerungen, insbesondere zum Erstellen von Zell-Sphäroiden und zum Überwachen des Zustands der Zell-Sphäroiden.

9. Verfahren zum markerfreien Erkennen und Klassifizieren von Zelländerungen, insbesondere zum Erstellen und Charakterisieren von Zell-Sphäroiden und zum Überwachen des Zustands der Zell-Sphäroiden in Echtzeit, Folgendes umfassend:
a) Bereitstellen einer Vorrichtung nach einem der Ansprüche 1 bis 4 und einer Kultivierungskammerplatte (1) nach einem der Ansprüche 5 bis 7,
b) Einbringen von Zellen, Zell-Sphäroiden oder einer Gewebeprobe in die Kulturreservoirs der Kultivierungskammerplatte (1),
c) Positionieren der Zellen, der Zell-Sphäroiden oder einer Gewebeprobe in den Mikrokavitäten (8) der Kultivierungskammerplatte (1) durch Bewegen des Orbitalschüttlers (3),
d) Bestimmen der Impedanz der Zellen, der Zell-Sphäroiden oder der Gewebeprobe zwischen zwei Mikroelektroden (10) und/oder elektrogener Aktivität der Zellen, der Zell-Sphäroiden oder der Gewebeprobe an jeder Mikroelektrode (10) und deren Änderungen im Laufe der Kultivierung und zwischen verschiedenen Kulturreservoirs.

10. Verfahren nach Anspruch 9 zum Erstellen und Charakterisieren von Zell-Sphäroiden und zum Überwachen des Zustands der Zell-Sphäroiden in Echtzeit, Folgendes umfassend:
- in Schritt b) werden Zellen in die Kulturreservoirs der Kultivierungskammerplatte (1) eingebracht,
- in einem weiteren Schritt b') werden Zell-Sphäroide durch Bewegungen des Orbitalschüttlers (3) erstellt und über zellspezifische Schüttelprogramme durch die Steuereinheit (6) verwaltet und
- in Schritt c) werden die Zell-Sphäroide in den Mikrokavitäten der Kulturreservoirs positioniert
- in Schritt d) wird die Impedanz der Gewebeprobe zwischen zwei Mikroelektroden (10) und/oder die elektrogene Aktivität der Zell-Sphäroiden an jeder Mikroelektrode (10) sowie ihre Änderungen im Laufe der Kultivierung und zwischen verschiedenen Kulturreservoirs bestimmt.

11. Verfahren nach Anspruch 9 oder 10, wobei ein bekannter oder vermuteter Modulator des Zellzustands in einer Teilmenge von Kulturreservoirs nach Schritt b), b') oder nach Schritt d) zugeführt wird.

12. Verfahren nach Anspruch 9 oder 10, wobei ein bekannter oder vermuteter Modulator des Zellzustands in einer Teilmenge von Kulturreservoirs nach Schritt d) zugeführt wird, ferner umfassend das iterative Wiederholen der Schritte c) und d).

13. Verfahren nach einem der Ansprüche 9 bis 12, wobei der Modulator eine möglicherweise toxische oder zytostatische Substanz und/oder ein pharmazeutischer Wirkstoff ist.

14. Verfahren nach einem der Ansprüche 10 bis 13, wobei die Zellen Stammzellen sind und das Erstellen der Zell-Sphäroiden eine Differenzierung der Stammzellen einleitet.

15. Verfahren nach Anspruch 14, wobei die Stammzellen in Kardiomyozyten differenzieren und der Modulator ein bekannter oder vermuteter Modulator der elektrophysiologischen Eigenschaften der Kardiomyozyten ist.

## Revendications

1. Dispositif de culture et de mesure intégré pour la détection sans marqueur et la classification de modifications cellulaires, pour la génération et la caractérisation de sphéroïdes cellulaires et pour la surveillance de l'état des sphéroïdes cellulaires en temps réel, comprenant
a) un dispositif de montage pour une plaque de chambre de culture (1), où la plaque de chambre de culture (1) comprend plusieurs réservoirs de culture, où le fond de chaque réservoir de culture forme une microcavité (8) et chaque microcavité (8) présente des microélectrodes (10) sur les parois de la microcavité (13) et où le dispositif de montage comprend des contacts pour les microélectrodes,
b) une carte d'amplificateur (2) reliée aux contacts pour les microélectrodes (10) dans le dispositif de montage,
c) un agitateur rotatif (3), sur lequel la carte d'amplificateur (2) et le dispositif de montage pour la plaque de chambre de culture (1) sont placés, et
d) une unité de commande (6), qui est reliée à la carte d'amplificateur (2) et à l'agitateur rotatif (3), où l'unité de commande (6) permet l'enregistrement, l'analyse de données et la commande du mouvement de l'agitateur rotatif (3).

2. Dispositif selon la revendication 1, élaboré de sorte que le positionnement de sphéroïdes cellulaires dans les microcavités (8) est géré automatiquement par l'unité de commande (6) grâce au mouvement de la carte d'amplificateur (2) et de la plaque de chambre de culture (1) par l'agitateur rotatif (3).

3. Dispositif selon la revendication 1 ou 2, comprenant en outre différents protocoles de rotation réglables pour l'agitateur rotatif (3) pour la génération de sphéroïdes cellulaires à partir de différents types de cellules et de tissus gérés par l'unité de commande (6).

4. Dispositif selon l'une des revendications 1 à 3, où le dispositif de montage pour la plaque de chambre de culture (1) est couplé à un système de manipulation microlaser.

5. Utilisation d'une plaque de chambre de culture (1) dotée de plusieurs réservoirs de culture dans un dispositif selon l'une des revendications 1 à 4, où le fond de chaque réservoir de culture forme une microcavité (8) et chaque microcavité (8) présente des microélectrodes (10) sur les parois de la microcavité (13).

6. Utilisation selon la revendication 5, où les microcavités (8) de la plaque de chambre de culture (1) sont conçues comme
a) une structure pyramidale tronquée inversée, ayant de préférence 4 à 8 côtés et une microélectrode (10) sur chaque côté, ou
b) une microcavité conique tronquée inversée, ayant de préférence 4 à 12 microélectrodes (10) sur la paroi (13).

7. Utilisation selon la revendication 5 ou 6, où la plaque de chambre de culture (1) présente un circuit intégré pour le prétraitement des données mesurées.

8. Utilisation d'un dispositif selon l'une quelconque des revendications 1 à 5 ou d'une plaque de chambre de culture telle que définie dans l'une quelconque des revendications 5 à 7 pour la culture de cellules ou de tissus, la détection sans marqueur et la classification de modifications cellulaires, en particulier pour la génération de sphéroïdes cellulaires et la surveillance de l'état de sphéroïdes cellulaires.

9. Procédé de détection sans marqueur et de classification de modifications cellulaires, en particulier pour la génération et la caractérisation de sphéroïdes cellulaires et pour la surveillance de l'état de sphéroïdes cellulaires en temps réel, comprenant :
a) la fourniture d'un dispositif selon l'une des revendications 1 à 4 et d'une plaque de chambre de culture (1) telle que définie dans l'une quelconque des revendications 5 à 7,
b) l'introduction de cellules, de sphéroïdes cellulaires ou d'un échantillon de tissu dans les réservoirs de culture de la plaque de chambre de culture (1),
c) le positionnement des cellules, des sphéroïdes cellulaires ou d'un échantillon de tissu dans les microcavités (8) de la plaque de chambre de culture (1) grâce au mouvement de l'agitateur rotatif (3),
d) la détermination de l'impédance des cellules, des sphéroïdes cellulaires ou d'un échantillon de tissu entre deux microélectrodes (10) et/ou de l'activité électrogène des cellules, des sphéroïdes cellulaires ou d'un échantillon de tissu sur chaque microélectrode (10) et de leurs changements pendant la culture et entre différents réservoirs de culture.

10. Procédé selon la revendication 9, pour la génération et la caractérisation de sphéroïdes cellulaires et pour la surveillance de l'état de sphéroïdes cellulaires en temps réel, comprenant:
- dans l'étape b), l'introduction des cellules dans les réservoirs de culture de la plaque de chambre de culture (1),
- dans une étape b') supplémentaire, la génération de sphéroïdes cellulaires grâce aux mouvements de l'agitateur rotatif (3) et leur gestion par l'unité de commande (6) via des programmes d'agitation spécifiques aux cellules et
- dans l'étape c), le positionnement des sphéroïdes cellulaires dans les microcavités des réservoirs de culture,
- dans l'étape d), la détermination de l'impédance de l'échantillon de tissu entre deux microélectrodes (10) et/ou de l'activité électrogène du sphéroïde cellulaire sur chaque microélectrode (10) et de leurs changements pendant la culture et entre différents réservoirs de culture.

11. Procédé selon la revendication 9 ou 10, dans lequel un modulateur connu ou présumé de l'état cellulaire dans un sous-ensemble de réservoirs de culture est introduit après l'étape b), b') ou après l'étape d).

12. Procédé selon la revendication 9 ou 10, dans lequel un modulateur connu ou présumé de l'état cellulaire dans un sous-ensemble de réservoirs de culture est introduit après l'étape d), comprenant en outre la répétition itérative des étapes c) et d).

13. Procédé selon l'une des revendications 9 à 12, dans lequel le modulateur est une substance éventuellement toxique ou cytostatique et/ou une substance active pharmaceutique.

14. Procédé selon l'une des revendications 10 à 13, dans lequel les cellules sont des cellules souches et la génération des sphéroïdes cellulaires initie une différentiation des cellules souches.

15. Procédé selon la revendication 14, dans lequel les cellules souches se différencient en cadiomyocytes et le modulateur est un modulateur connu ou présumé des propriétés électrophysiologiques des cardiomyocytes.
